# EUROPEAN PATENT APPLICATION

(11) **EP 2 564 835 A1**
(43) Date of publication of application: **06.03.2013**
(21) Application number: 11750099.1
(22) Date of filing: 01.03.2011
(51) Int. Cl.: A61K 9/20, A61K 31/165, A61P 31/04, A61K 47/02, A61K 47/34, A61K 47/26, A61K 47/30

(54) **FAST-DISSOLVING SOLID PHARMACEUTICAL FORM FOR TREATING BACTERIAL INFECTIONS**

(30) Priority: 01.03.2010 BR PI1002023
(71) Applicant: Nanocore Biotecnologia S.A., Campinas - SP, Cep: 13069-380 (BR)
(72) Inventor: RODRIGUES JR., José Maciel, 13107-110 Campina São Paulo (BR); LIMA, Karla de Melo, 13107-110 Campina São Paulo (BR)
(74) Representative: Fritz, Edmund Lothar
(86) International application number: PCT/BR2011/000054
(87) International publication number: WO 2011/106858

(57) **Abstract**

The present invention relates to solid pharmaceutical compositions that dissolve readily and stably and which are capable of inhibiting or eliminating an infectious process caused by bacteria in warm-blooded animals and aquatic species such as, for example, fish and crustaceans. The invention describes composition for formulations containing antibiotics to be administered by various routes for treating bacterial infections in warm-blooded animals and aquatic animals. The formulation can be intended for human and animal use.

## Description

### Field of the Invention

The present invention relates to solid pharmaceutical compositions that dissolve readily and stably and which are capable of inhibiting or eliminating an infectious process caused by bacteria in warm-blooded animals and aquatic species such as, for example, fish and crustaceans. The invention describes composition for formulations containing antibiotics to be administered by various routes for treating bacterial infections in warm-blooded animals and aquatic animals. The formulation can be intended for human and animal use.

### Background of Invention

The use of antibiotics to treat infections is of great economic relevance and impact on human and animal health. In food animals it is of great relevance to prevent and treat different types of infection in warm-blooded animals, either in cattle herd, goats, sheep, poultry and pigs and also for the treatment of pet animals such as cats, dogs and horses and also in pisciculture and carciniculture. The use of therapy with antibiotics wins even more importance for infections for which there are no prophylactic methods.

In veterinary practice, these products can be administered directly to individuals through pharmaceutical forms or can be added to food or drinking water, especially in the production of poultry and pigs. 0 use of this practice has been increasingly important for animals in confinement where the incidence of respiratory diseases is more common.

The main causative agents of respiratory infections in these species of production are of the genera Pasteurella, Haemophilus, Streptococcus, Bordetella, Salmonella, Actinobacillus and also Mycoplasma.

The most commonly used chemotherapeutic agents are intended to combat the infection which, among other symptoms, resulting in local and systemic inflammation, damage to lung tissue, fever and general morbidity with reduced immune response and, consequently, association with other infections especially from fungal origin, thus making the introduction of antibiotic treatment a priority.

Among the antibiotics used in veterinary medicine are the aminocyclitols (spectinomycin to apramycin), aminoglycosides (gentamicin and neomycin), beta-lactams (penicillins, amoxicillin, ceftiofur and cefarin), fluoroquinolones (enrofloxacin, ciprofloxacin and danofloxacin), the lincosamides (lincomycin, clindamycin and pirlimycin), macrolides (erythromycin, tilmicosin, tylosin), sulfonamides (combined or not), tetracyclines (tetracycline, chlortetracycline and oxytetracycline) and amphenicols (florfenicol and thiamphenicol).

The latter interferes with protein synthesis and gain more and more interest in medical practice because they are effective against gram negative and gram positive bacteria which cause respiratory infections. Because they are insoluble in water in usual concentrations, few formulations ready for use are available and there is no solid formulation available in major veterinary market in Brazil, intended for dilution in drinking water that would be useful, especially for poultry and pigs.

This is due to the low solubility of the amphenicols in water and has been the motivation for several studies to increase the solubility of drugs. The florfenicol, for example, presents, in addition to the low solubility in water (about 1.2 mg/mL), low wettability and, in contact with water, floats on the surface of the liquid which means that it is not completely solubilized. Only about 20% of the drug solubilises in water without addition of surfactants. To overcome this problem it has been proposed the use of pro-drugs as derivatives of amphenicols (which include derivatives of chloramphenicol, thiamphenicol and florfenicol) (US 4,311,857; US 4,582,918; US 4,973,750; US 4,876,352; US 5,227,494; US 4,743,700; US 5,567,844; US 5,105,009; US 5,382,673; US 5, 352,832; and US 5,663,361).

The US Patent 7,122,198 describes the use of encapsulation and association with surfactants using organic solvents in the process. Usually these production processes are complex, expensive, and often result in products of low shelf stability.

The patent application PCT/EP2004/008587 claims suspensions of micronised florfenicol. US patent 5,082,863; WO 2004/110494; WO 03028648; F R 0709197; and WO 2006/067138 describe liquid pharmaceutical compositions to be diluted in drinking water or to be used via injectable route ready for use.

In the pharmaceutical market it is found formulations available in a concentration of 30% with high viscosity which makes difficult the handling of the animals at the moment of injectable application by having poor conditions of syringeability. These formulations are not recommended for dilution in drinking water which makes difficult their use in poultry species whose injectable route is not desirable.

Other products offer a mixture of florfenicol as a pre-mixture (PREMIX) in feed for poultry and pork as well as an additive for food for fish, however, these products are not soluble in water.

An example of this effort refers to the patent application WO2008/085310 that claims a effervescent formulation through the combination of acidic and basic salts that react in contact with water allowing the solubility of the drug.

The patent application WO2009/061780 describes the association of florfenicol to polyvinylpyrrolidone (PVP) which is a hygroscopic product that enables the solubilization of florfenicol in a period of 15 minutes when added to water. The PVP has an important role in the composition because in his absence, only about 40% of the drug is solubilized in water. Irrespective the concentration of the drug, PVP and proposed diluent, the solubilization takes 15 minutes to be achieved which makes difficult its use if we consider that in practice the dissolution volumes are high as, for example, the use of water tanks with volumes exceeding 1,000 liters. To obtain the formulation, the PVP is diluted in water and the solution is used for wetting the granules and therefore it requires a process of drying at 50°C for 3 hours.

The patent application EP1480622 describes a immediate release composition of water-insoluble drugs. Such drugs are, for example, the florfenicol containing necessarily maltodextrin and a mixture of polyethylene glycols of high and low molecular weights.

Thus we see that various efforts have been published to make the solubility of drugs, including derived from amphenicols, and then solving this problem.

However, there is no solid product available on the market which can be release in the form of soluble power, which can be diluted at the time of use in drinking water or, alternatively, can be added to food in solid form and after it is ingested it will be quickly solubilized in the gastrointestinal tract, allowing effective absorption by the body.

### Summary of the invention

Surprisingly, the present invention describes a solid product which is soluble in water without addition of PVP or water during the process of its preparation. The product of the present invention contains a derived of amphenicol or mixture thereof which in contact with water promotes the solubility of amphenicol in just a minute. More than 90% of the drug is solubilized immediately when it is in a concentration of up to 10% w/w in the formulation (less than a minute) and more than 90% up to 3 minutes in the concentration of 25% w/w. The preparation of the product is simple without intermediate processes that require product exposure to high temperatures. In addition, there is no addition of water for wetting and there is no need for expensive processes of formulation such as fluidized bed granulation. The product is obtained by dry route through simple mixture of constituents.

In the present invention it is reported a solid formulation, soluble in water, which can be added directly to the drinking water of warm-blooded animals and can also be added to food at the time of treatment or can be still added to feed during the pelleting process. Alternatively, the formulation can be previously dissolved in water and release in the form of a solution for dilution in drinking water.

The present invention describes formulations in the form of fluid powder soluble in water, said fluid power being composed of a compound derived from amphenicol in a concentration of amphenicol from 1% to 50% w/w, preferably in the range of 10% to 25% w/w. The formulation composed of a diluent that can be lactose and low weight polyethylene glycol (PEG 400) and a surfactant selected from the group of polysorbate for example Tween and, alternatively, an adjuvant to increase the fluidity of the powder. In contact with water the product dissolves quickly around 1 minute.

### Brief description of the drawings

Figure 1 shows the chemical structure of amphenicol and its main clinical interest derivatives in combating bacterial infections.
Figure 2 shows the chemical structure of florfenicol.
Figure 3 illustrates the dissolution profile of florfenicol using different preparations in water.

### Detailed Description of the Invention

In the present invention were developed stable pharmaceutical compositions containing antibiotics for the treatment of infections in warm-blooded animals, for human or animal use, preferably for the treatment of respiratory infections in humans or in food animals. In the latter case the composition is to be added in drinking water or food.

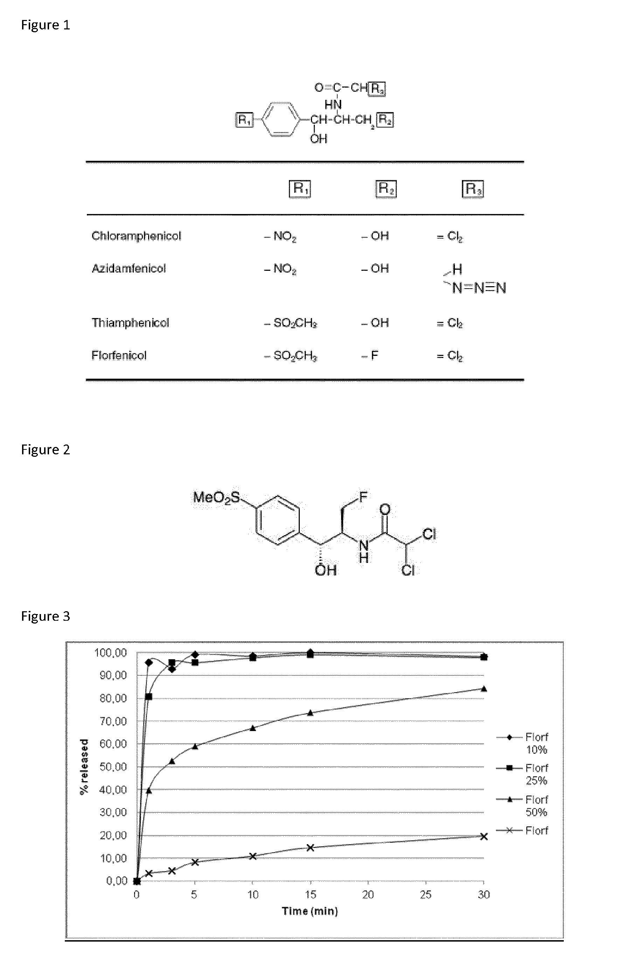

Pharmaceutical composition for the treatment of infections in warm-blooded animals, object of the present invention comprises:
(a) at least one drug selected from the group of amphenicol antibiotics (Figure 1), preferably the florfenicol (Figure 2), or mixture of amphenicol;
(b) at least one water-soluble vehicle selected from the group of sugars such as lactose at a concentration of 20 to 83%;
(c) a co-solvent that should also act as an wetting agent selected from the group of polyalcohols, preferably from the group of polyethylene glycol of low molecular weight in a concentration between 0.1 to 10%, preferably between 2 and 4%;
(d) a surfactant agent selected from the group of polysorbates in a concentration between 0.1 to 10%, preferably between 3 and 7%.

More preferred, in order to obtain a product with enhanced fluidity it can be optionally added:
(e) an adjuvant which increases the fluidity of powder, said adjuvant being preferentially a colloidal type.

The optional adjuvant used to increase the fluidity is preferably the colloidal silicon dioxide.

The composition presents improved fluidity and can be used in solid dosage form known from the state of the art and also in a liquid form for dilution or as a sterile powder for dilution at the time of use.

| | | | |
|---|---|---|---|
| 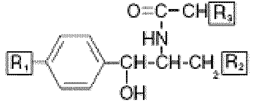 | | | |

| | 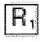 | 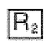 | 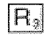 |
|---|---|---|---|
| Chloramphenicol | - NO₂ | - OH | = Cl₂ |
| Azidamfenicol | - NO₂ | -OH | 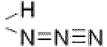 |
| Thiamphenicol | - SO₂CH₃ | - OH | = Cl₂ |
| Florianicol | - SO₂CH₃ | - F | = Cl₂ |

### Figure 2 - Chemical structure of florfenicol

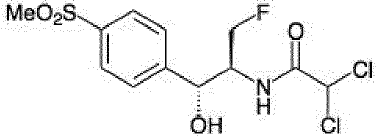

The object of the present invention can be best described by the following examples, which should not be considered as limiting the scope of protection.

### Example 1

| **Ingredients** | **Concentration (100 grams)** |
|---|---|
| Florfenicol | 10g |
| Lactose | 81.8g |
| PEG 400 | 3.0g |
| Tween 80 | 3.7g |
| colloidal silicon dioxide | 1.5g |

The object of the present invention can be best described by the following examples, which should not be considered as limiting the scope of protection.

### Example 1

| **Ingredients** | **Concentration (100 grams)** |
|---|---|
| Florfenicol | 10g |
| Lactose | 81.8g |
| PEG 400 | 3.0g |
| Tween 80 | 3.7g |
| colloidal silicon dioxide | 1.5g |

The ingredients can be mixed in a single step. Preferably, the florfenicol was mixed with lactose and with aerosol until a complete homogenization. Then, the wetting of the powders was carried out through the slowly addition of a granulating solution containing Tween and PEG 400. The mixture was completed homogenized. The final product can be calibrated using tamis and the product can be packed in plastic or glass or paper or aluminized packages. Optionally this formulation can be added to proteic concentrates, such as fish feed in the form of pelletized or grainy.

### Example 2

| **Ingredient** | **Concentration (100 grams)** |
|---|---|
| Florfenicol | 25g |
| Lactose | 64.8g |
| PEG 400 | 3.9g |
| Tween 80 | 4.8g |
| colloidal silicon dioxide | 1.5g |

The ingredients can be mixed in a single step. Preferably, the florfenicol was added to lactose and to aerosil until complete homogenization. Then, the wetting of the powders was carried out through the slowly addition of a granulating solution containing Tween and PEG 400. The mixture was completed homogenized.

The final product can be calibrated using tamis and the product can be packed in plastic or glass or paper or aluminized packages.

Optionally this formulation can be added to proteic concentrates, such as fish feed in the form of pelletized or grainy.

### Example 3

| **Ingredient** | **Concentration (100 grams)** |
|---|---|
| Florfenicol | 50g |
| Lactose | 38.2g |
| PEG 400 | 4.6g |
| Tween 80 | 5.7g |
| colloidal silicon dioxide | 1.5g |

The ingredients can be mixed in a single step. Preferably, the florfenicol was added to lactose and to aerosil until complete homogenization. Then, the wetting of the powders was carried out through the slowly addition of a granulating solution containing Tween and PEG 400. The mixture was completed homogenized. The final product can be calibrated using tamis and the product can be packed in plastic or glass or paper or aluminized packages. Optionally this formulation can be added to proteic concentrates, such as fish feed in the form of pelletized or grainy

### Example 4

| **Ingredient** | **Concentration (100 grams)** |
|---|---|
| Tianfenicol | 10g |
| Lactose | 80.8g |
| PEG 400 | 3.5g |
| Tween 80 | 4.2g |
| colloidal silicon dioxide | 1.5g |

The ingredients can be mixed in a single step. Preferably, the florfenicol was added to lactose and to aerosil until complete homogenization. Then, the wetting of the powders was carried out through the slowly addition of a granulating solution containing Tween and PEG 400. The mixture was completed homogenized. The final product can be calibrated using tamis and the product can be packed in plastic or glass or paper or aluminized packages. Optionally this formulation can be added to proteic concentrates, such as fish feed in the form of pelletized or grainy

### Example 5: Dissolution profile of florfenicol in water

The dissolution test was conducted in order to obtain a final concentration of florfenicol of 0.02 mg/mL in water and determine the optimal concentration of the drug in the formulation. Thus, 3 formulations at concentrations of 10; 25; and 50%, respectively were prepared. For the dissolution test, it was weighted 200 mg, 80 mg and 40 mg from the formulations of florfenicol 10% soluble powder; florfenicol 25% soluble powder and florfenicol 50% soluble powder, respectively. Dilution was made in Milli Q water and for assessment of diluted percentage, samples of 1 m L were collected at the following time 0, 1, 3, 5, 10, 15 and 30 minutes. The samples were immediately filtered in a 0.45 mm filter and stored for analysis of florfenicol. To perform the test it was used a Pharma Test dissolutor (Germany) model 70 PTDT type shovel with a volume 1000 mL of Milli Q water, stirring 100 rpm and temperature of 25°C. The analyses of the contents of florfenicol were performed using liquid chromatograph of high efficiency model LC 2010C (Shimadzu ®, Japan) with UV detection at a wavelength of 224 nm, chromatographic column C8 150 mm x 4.60 mm, 5µm (Phenomenex ®) at the temperature of 40°C, mobile phase comprising acetonitrile:sodium acetate 0.01 mol/L, pH 4.4 in the ratio of 40:60 (v/v), mobile phase flow of 1.5 mL/min and 20 mL injection volume. The results are illustrated in Figure 3.

From these results we conclude that formulations at concentrations between 10 and 25% are easily dissolved in 1 and 3 minutes, respectively. This time is much lower than the time known from the state of the art as described in WO2009/061780 which is around 15 minutes. This data is of great importance if we consider the form of application. Another relevant fact is that the formulation of the present invention there is no need to add polyvinylpyrrolidone.

## Claims

1. A pharmaceutical composition for treatment of infections in warm-blooded animals and aquatic species comprising:
(a) at least one drug selected from the group of amphenicol antibiotics or mixture of amphenicols;
(b) at least one carrier selected from the group of sugars in concentrations between 20% to 83%, preferably between 60 to 82%;
(c) a polyalcohol of low molecular weight;
(d) a surfactant from the group of polysorbates.

2. Composition according to claim 1 wherein the drug from the group of amphenicol be florfenicol.

3. Composition according to claim 1 wherein the drug from the group of amphenicol be thiamphenicol.

4. Composition according to claim 2 wherein the florfenicol is in diluted in the formulation at a concentration of 1% to 50% w/w, preferably 10% to 25% w/w.

5. Composition according to claim 3 wherein thiamphenicol is diluted at a concentration of 1% to 50% w/w, preferably 10% to 25% w/w, more preferably 10% w/w.

6. Composition according to claim 1 wherein the carrier is lactose.

7. Composition according to claim 6 wherein the concentration of lactose is between 20% and 83% w/w.

8. Composition according to claim 1 wherein the polyalcohol is a low molecular weight polyethylene glycol (PEG 400).

9. Composition according to claim 1 wherein the surfactant is polysorbate 80.

10. Composition according to claim 1 wherein an adjuvant is added to increase the fluidity of the powder.

11. Composition according to claim 11 wherein the adjuvant is colloidal silicon dioxide.

12. Composition according to any one of claims 1-11 wherein the dissolution of the product is in water and released in the form of solution, preferably at a concentration between 10 and 25%.

13. Method of treating warm-blooded animals and aquatic species **characterized by** the dissolution of the composition according to claims 1 to 12 in water at concentrations of 0.01 mg/mL to 0.20 mg/mL.
